# EUROPEAN PATENT SPECIFICATION

(11) **EP 3 363 779 B1**
(45) Date of publication and mention of the grant of the patent: **22.09.2021**
(21) Application number: 16868560.0
(22) Date of filing: 22.11.2016
(51) Int. Cl.: C07C 68/02, C07C 69/96

(54) **PRODUCTION METHOD OF ASYMMETRIC CHAIN CARBONATE**
HERSTELLUNGSVERFAHREN FÜR ASYMMETRISCHES KETTENCARBONAT
PROCÉDÉ DE PRODUCTION DE CARBONATE À CHAÎNE ASYMÉTRIQUE

(30) Priority: 24.11.2015 JP 2015229018
(43) Date of publication of application: 22.08.2018
(73) Proprietor: DAIKIN INDUSTRIES, LTD., Osaka-shi, Osaka 530-8323 (JP)
(72) Inventor: HAMADA, Tomohito, Osaka 530-8323 (JP); OKADA, Michiaki, Osaka 530-8323 (JP); YAMAUCHI, Akiyoshi, Osaka 530-8323 (JP); KISHIKAWA, Yosuke, Osaka 530-8323 (JP)
(74) Representative: Hoffmann Eitle
(86) International application number: PCT/JP2016/084621
(87) International publication number: WO 2017/090617

(56) References cited:
- EP-A1- 2 380 868
- WO-A1-2004/024658
- WO-A1-2006/054833
- WO-A1-2015/083745
- WO-A1-2015/083747
- GB-A- 629 019
- GB-A- 2 067 194
- JP-A- S6 248 650
- US-A1- 2012 141 870
- US-A1- 2012 141 870
- ZHEN-ZHEN YANG ET AL: "Dimethyl carbonate synthesis catalyzed by DABCO-derived basic ionic liquids via transesterification of ethylene carbonate with methanol", TETRAHEDRON LETTERS, vol. 51, no. 21, 1 May 2010 (2010-05-01), pages 2931-2934, XP055555726, AMSTERDAM, NL ISSN: 0040-4039, DOI: 10.1016/j.tetlet.2010.03.114
- WANG SONGLIN ET AL: "Preparation and catalytic property of MoO3/SiO2for disproportionation of methyl phenyl carbonate to diphenyl carbonate", JOURNAL OF MOLECULAR CATALYSIS A: CHEMICAL, vol. 398, 24 November 2014 (2014-11-24), pages 248-254, XP029143649, ISSN: 1381-1169, DOI: 10.1016/J.MOLCATA.2014.11.014
- JAROMÍR MINDL ET AL: "Alkoxycarbonylation of Alcohols and Phenols by Nitrosoformates", COLLECTION SYMPOSIUM SERIES (XIIITH SYMPOSIUM ON CHEMISTRY OF NUCLEIC ACID COMPONENTS SPINDLERUV MLYN, CZECH REPUBLIC; SEPTEMBER 03 -09, 2005), vol. 61, no. 7, 18 March 1996 (1996-03-18) , pages 1053-1063, XP055587639, CZ ISSN: 0010-0765, DOI: 10.1135/cccc19961053 ISBN: 978-80-86241-25-8

## Description

### TECHNICAL FIELD

The present invention relates to a method for producing an asymmetric chain carbonate. The present invention also relates to a composition containing an asymmetric carbonate.

### BACKGROUND ART

US 2012/0141870 reports production of methyl-2,2,2-trifluoroethyl carbonate with a yield of 46% by adding methyl chloroformate to a solution containing 2,2,2-trifluoroethanol, pyridine, and dichloromethane.

WO 2015/083745 and WO 2015/083747 describe production of highly purified methyl-2,2,2-trifluoroethyl carbonate by mixing trifluoroethanol, pyridine, and triglyme, and dropwise adding methyl chlorocarbonate to the mixture to synthesize methyl-2,2,2-trifluoroethyl carbonate, followed by rectification to collect highly purified methyl-2,2,2-trifluoroethyl carbonate.

Z.-Z. Yang et al., Tetrahedron Letters, 51, 2931-2934 (2010) reports on the synthesis of dimethyl carbonate by transesterification of ethylene carbonate with methanol, catalyzed by DABCO-derived basic ionic liquids, yielding mixtures containing both the symmetric dimethyl carbonate and the asymmetric 2-hydroxyethylmethyl carbonate.

S. Wang et al., J of Mol. Catalysis A, 398, 248-254 (2015) relates to the preparation and catalytic properties of MoO₃/SiO₂ for disproportionation of methyl phenyl carbonate to diphenyl carbonate. Dimethyl carbonate reacts in an equilibrium reaction with phenol to form the asymmetric methyl phenyl carbonate, and two MPC molecules can further react in a second equilibrium reaction to form DMC and diphenyl carbonate.

EP-A-2 380 868 describes a process for simultaneously producing a symmetric dialkyl carbonate and an asymmetric dialkyl carbonate by transesterification of an alkylene carbonate with two or more kinds of alcohols.

WO 2006/054833 discloses a method for preparing asymmetric linear carbonates by transesterification by reacting symmetric linear carbonates and linear ester compounds in the presence of a basic catalyst.

WO 2004/024658 relates to the catalytic conversion of organic carbonates to the corresponding alcohol by contacting organic carbonates with alcohol in the presence of a catalyst, yielding mixtures of symmetric and asymmetric carbonates.

J. Mindl et al., Coll. Symposium Series, 61(7), 1053-1063 (1996) describes the alkoxy carbonylation of alcohols and phenols by nitrosoformates, yielding mixtures of symmetric and asymmetric carbonates.

GB-A-629 019 discloses asymmetric chain carbonate, which are prepared by reacting an alcohol R-OH with a halocarbonate ester XCOOR' wherein R and R' are different organic groups and X is halogen in the presence of an inorganic base.

GB-A-2 067 194 refers to a method for preparing allyl carbonate monomer, wherein aqueous alkali metal hydroxide is added to an organic solution of polyol chloroformate and allyl alcohol, and recovering an organic phase containing the allyl carbonate monomer, and an aqueous phase containing diallyl carbonate and unreacted allyl alcohol.

US-A-2012/141870 relates to multifunctional sulfone/fluorinated ester solvents and discloses the preparation of the asymmetric methyl 2,2,3,3-tetrafluoropropyl carbonate and methyl 2,2,2-trifluoroethyl carbonate.

### SUMMARY OF INVENTION

### - Technical Problem

Unfortunately, the conversion rate and selectivity are insufficient in conventional techniques using pyridine.

In view of the current state of the art described above, the present invention aims to provide a method for producing an asymmetric chain carbonate by reacting an alcohol with a halocarbonate ester compound at a high conversion rate and high selectivity.

### - Solution to Problem

The present invention relates to a method for producing an asymmetric chain carbonate, comprising reacting an alcohol of the formula R¹OH (1) wherein R¹ is an organic group, with a halocarbonate ester compound of the formula XCOOR² (2) wherein X is a halogen atom; and R² is an organic group different from R¹, in the presence of a basic magnesium salt, or a basic alkaline earth metal salt, or an inorganic base and a desiccant, to produce an asymmetric chain carbonate of the formula R¹OCOOR² (3), wherein R¹ and R² are as defined above.

In the production method of the present invention ("the present method" hereinafter), preferably, R¹ is alkyl optionally having a fluorine atom, and R² is alkyl optionally having a fluorine atom.

### - Advantageous Effects of Invention

The present method with the above-described features enables production of an asymmetric chain carbonate by reacting an alcohol with a halocarbonate ester compound at a high conversion rate and high selectivity.

### DESCRIPTION OF EMBODIMENTS

The present invention will be specifically described below.

In a first embodiment of the invention, the present method is a method for producing an asymmetric chain carbonate of Formula (3) by reacting an alcohol of Formula (1) with a halocarbonate ester compound of Formula (2) in the presence of a basic magnesium salt or a basic alkaline earth metal salt. The present inventors have found that a high conversion rate and high selectivity can be surprisingly achieved using a basic magnesium salt or a basic alkaline earth metal salt.

Examples of the basic magnesium salt or basic alkaline earth metal salt include basic magnesium salts such as magnesium oxide, magnesium hydroxide, magnesium carbonate, magnesium hydrogen carbonate, and magnesium acetate; basic calcium salts such as calcium oxide, calcium hydroxide, calcium carbonate, calcium hydrogen carbonate, and calcium acetate; basic strontium salts such as strontium oxide, strontium hydroxide, strontium carbonate, strontium hydrogen carbonate, and strontium acetate; basic barium salts such as barium oxide, barium hydroxide, barium carbonate, barium hydrogen carbonate, and barium acetate; and mixtures thereof.

The basic magnesium salt or basic alkaline earth metal salt is preferably a basic calcium salt. Usually, a weak acid calcium salt is used. In particular, it is more preferably at least one selected from calcium oxide, calcium hydroxide, calcium carbonate, calcium hydrogen carbonate, and calcium acetate.

The amount of the basic calcium salt or basic alkaline earth metal salt per mol of the alcohol of Formula (1) is preferably 0.5-2.0 mol, - more preferably ≥ 0.7 mol and more preferably ≥ 1.5 mol. In the case of using two or more salts, the amount means the total amount of all the salts.

The purity of the basic calcium salt or basic alkaline earth metal salt is not particularly limited, but is preferably ≥ 90 mass-% to keep expected reactivity. Usually, no other salt is used, but other neutral or basic inorganic salts may be contained.

The calcium oxide may contain impurities such as hydrochloric acid-insoluble substances, chlorides, carbonates, sulfates, heavy metals, and iron, but a smaller amount is better. For example, acceptable amounts of impurities are as follows.
Hydrochloric acid-insoluble substance: ≤ 0.5%
Chloride (Cl): ≤ 0.25%
Carbonate: test-passed amount (forming almost no bubbles)
Sulfate (SO₄) : ≤ 1.0%
Heavy metal (Pb): ≤ 0.025%
Iron (Fe): ≤ 0.25%

The calcium oxide preferably has an ignition loss (800°C) of ≤ 6.0%. The calcium oxide content (after ignition) is preferably ≥ 98.0%.

The calcium hydroxide preferably has a purity of ≥ 96.0%. The calcium hydroxide may contain impurities such as hydrochloric acid-insoluble substances, carbonates, chlorides, sulfates, sodium, potassium, magnesium, lead, arsenic, chromium, manganese, and iron, but a smaller amount is better. For example, acceptable amounts of impurities are as follows.
Hydrochloric acid-insoluble substance: ≤ 0.5%
Carbonate: test-passed amount (forming almost no bubbles)
Chloride (Cl): ≤ 0.05%
Sulfate (SO₄) : ≤ 0.25%
Sodium (Na): ≤ 0.25%
Potassium (K): ≤ 0.25%
Magnesium (Mg): ≤ 5.0%
Lead (Pb): ≤ 0.015%
Arsenicum (As): 2.5 ppm
Chromium (Cr): ≤ 0.025%
Manganese (Mn): ≤ 0.05%
Iron (Fe): ≤ 0.10%

The calcium hydroxide preferably meets the standard prescribed in JIS K 8575-1994.

In the present first embodiment, the below-described desiccant is not essential, but may be used.

In a second embodiment of the invention, the production method is a method for producing an asymmetric chain carbonate of Formula (3) by reacting an alcohol of Formula (1) with a halocarbonate ester compound of Formula (2) in the presence of an inorganic base and a desiccant. The present inventors have found that a high conversion rate and high selectivity can be surprisingly achieved using an inorganic base and a desiccant.

Preferably, the inorganic base is at least one of carbonates of alkali metals, hydroxides of alkali metals, oxides of magnesium, oxides of alkaline earth metals, hydroxides of magnesium, hydroxides of alkaline earth metals, carbonates of magnesium, carbonates of alkaline earth metals, acetates of magnesium, and acetates of alkaline earth metals. More preferably, it is at least one of potassium carbonate, sodium carbonate, potassium hydroxide, and sodium hydroxide.

The amount of the inorganic base per mol of the alcohol of Formula (1) is preferably 0.5-2.0 mol, and is more preferably ≥ 0.7 mol and more preferably ≤ 1.5 mol.

The desiccant is preferably at least one of neutral calcium salts, zeolite, silica gel, alumina, and activated carbon, more preferably at least one of calcium chloride, zeolite, and alumina.

The neutral calcium salt usually means a strong acid calcium salt. It is preferably at least one of calcium chloride, calcium bromide, calcium iodide, and calcium sulfate.

The purity of the neutral calcium salt is not particularly limited, but is preferably ≥ 90 mass-% to keep expected reactivity. Usually, no other salt is used, but other neutral or basic inorganic salts may be contained.

Each neutral calcium salt may be used alone or in combination of two or more.

The neutral calcium salt may be activated before use. The activation is performed, for example, by drying treatment with heating at 300-350°C or at 350-400°C overnight in vacuum (10⁻¹ to 10⁻³ mmHg) . Neutral calcium salts not activated by such a treatment can also be suitably used.

The zeolite may be natural zeolite or a synthesized zeolite.

The zeolite is preferably a synthesized zeolite.

The zeolite is preferably one of the formula M_{2/n}O·Al₂O₃-xSiO₂·yH₂O (wherein M is a metal cation, n is the valence of the metal cation, x is a coefficient, and y is a coefficient). The metal cation as M is preferably at least one metal cation selected from a sodium cation, a lithium cation, and a potassium cation.

The zeolite is preferably porous.

The zeolite preferably has an average pore size of 0.3-0.5 nm (3-5 Å), more preferably 0.3-0.4 nm (3-4 Å).

The morphology of the zeolite may be, for example, powder, granule, or pellet, and is preferably powder or granule.

The zeolite has a weight average particle diameter of preferably ≤ 10 µm, more preferably ≤ 5 µm. Herein, the particle diameter refers to a major axis. Herein, in the case where the primary particles of zeolite form secondary particles, the term "weight average particle diameter" refers to the particle diameter of the secondary particles.

Each zeolite may be used alone or in combination of two or more.

The zeolite may be activated before use. The activation is performed, for example, by drying treatment with heating at 300-350°C overnight in vacuum (10⁻¹ to 10⁻³ mmHg). Zeolite not activated by such a treatment can also be suitably used.

The silica gel may be spherical or crushed silica gel.

In the case of spherical silica gel, the particle diameter thereof upon use does not matter, but is more preferably 40-500 µm.

The silica gel may be acid silica gel, neutral silica gel, or basic silica gel such as amino silica.

Each silica gel may be used alone or in combination of two or more.

The silica gel may be activated before use. The activation is performed, for example, by drying treatment with heating at 300-350°C overnight in vacuum (10⁻¹ to 10⁻³ mmHg). Silica gel not activated by such a treatment can also be suitably used.

Alumina having any shape may be used.

The particle diameter of the alumina upon use does not matter, but is preferably 50-100 µm.

The alumina may be acid alumina, neutral alumina, or basic alumina.

Each alumina may be used alone or in combination of two or more.

The alumina may be activated before use. The activation is performed, for example, by drying treatment with heating at 100-150°C overnight in vacuum (10⁻¹ to 10⁻³ mmHg). Almina not activated by such a treatment can also be suitably used.

The activated carbon may be crushed, granulated, or molded activated carbon.

In the case of spherical activated carbon, the particle diameter thereof does not matter, but is more preferably 50-100 µm.

The activated carbon may be gas-activated carbon, zinc chloride-activated carbon, or phosphoric acid-activated carbon.

Each activated carbon may be used alone or in combination of two or more.

The activated carbon may be activated before use. The activation is performed, for example, by firing at 800-1000°C. Activated carbon not having undergone such a treatment can also be suitably used.

The alcohol is represented by the formula R¹OH (1) wherein R¹ is an organic group.

Examples of the organic group include alkyl, alkenyl, alkynyl, cycloalkyl, cycloalkenyl, aryl and heteroaryl, each optionally having one or more substituent(s).

The organic group is preferably alkyl or aryl, each optionally having one or more substituent(s).

Examples of the substituent include halogen (preferably fluorine), alkyl, fluorinated alkyl, aryl, and heteroatom-containing groups. Preferred of these is halogen, and more preferred is fluorine. Examples of the heteroatom-containing groups include groups containing N, O, or S, such as an amino group, an amide group, a hydroxy group, an ether bond, an ester bond, a thiol bond, and a SH group.

The number of the substituents is preferably 1-4, more preferably 1-3, still more preferably 1 or 2.

The organic group is preferably an optionally substituted alkyl group.

The organic group may be alkyl optionally having a halogen atom, or may be alkyl optionally having a fluorine atom.

The organic group is more preferably alkyl having a halogen atom, still more preferably alkyl having a fluorine atom.

The organic group has preferably ≤ 20, more preferably ≤ 10 , still more preferably ≤ 5, particularly preferably ≤ 3, most preferably ≤ 2 carbon atoms, and has ≥ 1 carbon atom(s).

The alkyl group has preferably ≤ 20, more preferably ≤ 10 , still more preferably ≤ 5, particularly preferably ≤ 3, most preferably ≤ 2 carbon atoms, and has ≥ 1 carbon atom(s).

The alkyl group may be linear or branched.

Examples of R¹ include -CH₃, -CH₂CH₃, -CH₂-O-CH₃, -CH₂-CH₂-O-CH₃, -C₆H₅, -CF₃, -CF₂H, -CFH₂, -CH₂CF₃, -CH₂CF₂H, - CH₂CFH₂, -CF₂CF₃, -CF₂CF₂H, -CF₂CFH₂, -CFHCF₃, -CFHCF₂H, - CFHCFH₂, -CH(CF₃)₂, -CH₂C₂F₅, -CH₂CF₂CF₃, -CH₂CF₂CF₂H, - CF₂CF₂CF₃, -CHFCH₃, and -CF₂CH₃.

In particular, in order to achieve a high conversion rate and high selectivity, R¹ is preferably C₁₋₃-alkyl having a fluorine atom, more preferably at least one of - CF₃, -CF₂H, -CFH₂, -CH₂CF₃, -CH₂CF₂H, -CH₂CFH₂, -CF₂CF₃, - CF₂CF₂H, -CF₂CFH₂, -CFHCF₃, -CFHCF₂H, -CFHCFH₂, -CH(CF₃)₂, - CH₂C₂F₅, -CH₂CF₂CF₃, -CH₂CF₂CF₂H, and -CF₂CF₂CF₃, still more preferably at least one of -CH₂CF₃ and -CF₂CF₃.

The halocarbonate ester compound is represented by the Formula XCOOR² (2), wherein X is a halogen atom, and R² is an organic group different from R¹.

The halogen atom as X is preferably F, Cl, Br, or I, more preferably F or Cl, still more preferably Cl.

The halocarbonate ester compound to be used has a purity of 95-100 mass-%, and may be used without undergoing any treatment or after purified by a usual method such as dehydration or distillation. Unpurified halocarbonate ester compounds may also be suitably used.

R² is an organic group different from R¹.

Examples of the organic group include alkyl, alkenyl, alkynyl, cycloalkyl, cycloalkenyl, aryl and heteroaryl, each optionally having one or more substituent(s).

The organic group as R² is preferably alkyl or aryl, each optionally having one or more substituent(s).

Examples of the substituent include halogen (preferably fluorine), alkyl, fluorinated alkyl, aryl, and heteroatom-containing groups. Examples of heteroatom-containing groups include groups containing N, O, or S, such as an amino group, an amide group, a hydroxy group, an ether bond, an ester bond, a thiol bond, and a SH group.

The number of the substituents is preferably 1-4, more preferably 1-3.

The organic group as R² is preferably optionally substituted alkyl.

The organic group as R² may be alkyl optionally having a halogen atom, or may be alkyl optionally having a fluorine atom.

The organic group as R² has preferably ≤ 20, more preferably ≤ 10 , still more preferably ≤ 5, particularly preferably ≤ 3, most preferably ≤ 2 carbon atoms, and has ≥ 1 carbon atom(s).

The alkyl group has preferably ≤ 20, more preferably ≤ 10 , still more preferably ≤ 5, particularly preferably ≤ 3, most preferably ≤ 2 carbon atoms, and has ≥ 1 carbon atom(s) .

The alkyl group may be linear or branched.

Examples of R² include -CH₃, -CH₂CH₃, -CH(CH₃)₂, - CH₂CF₃, and -CH₂C₂H₅. In particular, in order to achieve a high conversion rate and high selectivity, R² is preferably C₁₋₃-alkyl having no fluorine atom, more preferably at least one of - CH₃, -CH₂CH₃, -CH(CH₃)₂, and -CH₂C₂H₅, still more preferably -CH₃.

To achieve a high conversion rate and high selectivity, preferably at least one of R¹ and R² is alkyl having a halogen atom. More preferably, one of R¹ and R² is alkyl having a halogen atom, and the other is alkyl having no halogen atom.

To achieve a high conversion rate and high selectivity, preferably at least one of R¹ and R² is alkyl having a fluorine atom. More preferably, one of R¹ and R² is alkyl having a fluorine atom, and the other is alkyl having no fluorine atom.

The asymmetric chain carbonate is represented by the Formula R¹OCOOR² (3), wherein R¹ and R² are as defined above.

The present method is particularly suitable to produce an asymmetric chain carbonate such as CF₃OCOOCH₃, CF₃CH₂OCOOCH₃, CF₃CF₂OCOOCH₃, CF₃CF₂CF₂OCOOCH₃, CF₃CF(CF₃)OCOOCH₃, CF₃CH(CF₃)OCOOCH₃, CF₃CF(CH₃)OCOOCH₃, CF₃CH(CH₃)OCOOCH₃, CF₃OCOOCH₂CH₃, CF₃CH₂OCOOCH₂CH₃, CF₃CF(CF₃)OCOOCH₂CH₃, CF₃CH(CF₃)OCOOCH₂CH₃, CF₃CF(CH₃)OCOOCH₂CH₃, CF₃CH(CH₃)OCOOCH₂CH₃, CH₃OCOOCFHCH₃, and CH₃CH₂OCOOCFHCH₃.

The reactions in the present method may be performed in a solvent, but use of a solvent is not essential. Use of a solvent is advantageous in facilitating mixing of raw materials and removal of heat generated in the reactions. Use of no solvent is advantageous in simplifying collection of target products and cost efficiency.

The solvent is preferably an organic solvent. Use of water as a solvent may lead to an insufficient conversion rate.

Examples of the solvent include esters such as methyl acetate, ethyl acetate, propyl acetate, n-butyl acetate, and tert-butyl acetate; ketones such as acetone, methyl ethyl ketone, and cyclohexanone; aliphatic hydrocarbons such as hexane, cyclohexane, octane, nonane, decane, undecane, dodecane, and mineral spirits; aromatic hydrocarbons such as benzene, toluene, xylene, naphthalene, and solvent naphtha; alcohol such as methanol, ethanol, tert-butanol, iso-propanol, and ethyleneglycol monoalkyl ethers; chain ethers such as dimethyl ether and diethyl ether; cyclic ethers such as tetrahydrofuran, tetrahydropyran, and dioxane; halogenated hydrocarbons such as chloromethane, dichloromethane, trichloromethane (chloroform), tetrachloromethane, 1,2-dichloroethane, and difluoroethane; amides such as N-methylpyrrolidone, dimethylformamide, and dimethylacetamide; nitriles such as acetonitrile; and sulfoxides such as dimethyl sulfoxide.

In particular, the solvent is preferably at least one of dodecane, octane, nonane, decane, dichloromethane, 1,2-dichloroethane, chloroform, carbon tetrachloride, toluene, xylene, ethyl acetate, propyl acetate, and butyl acetate. If the target carbonate is liquid, such a solvent is preferred in view of simplification of the reaction system.

The reaction temperature is preferably -20°C to 40°C, more preferably -10°C to 30°C, still more preferably 0°C to 20°C. At too high a reaction temperature, the halocarbonate ester compound of Formula (2) may be deactivated.

The duration of the reaction is preferably 0.1-100 hours, more preferably 1-90 hours, still more preferably 5-80 hours.

The reaction may be performed at atmospheric pressure or elevated pressure, for example, at 1 MPa (gauge pressure) or lower.

The present method may further include distillation of the asymmetric chain carbonate produced by the reaction.

A composition that can be produced by the present method includes:
an asymmetric chain carbonate of the Formula R¹OCOOR² (3), wherein R¹ is an organic group; and R² is an organic group different from R¹, and at least one of symmetric chain carbonates of the Formulae R¹OCOOR¹ (4) and R²OCOOR² (5), wherein R¹ and R² are as defined above.

The amount of the asymmetric chain carbonate of Formula (3) relative to the composition is preferably 30-99.99 mass-%, more preferably 50-99.95 mass-%, still more preferably 80-99.9 mass-%.

The amount of the symmetric chain carbonate of Formula (4) relative to the composition is preferably 0.01-10 mass-%, more preferably 0.05-5 mass-%, still more preferably 0.1-2 mass-%.

The amount of the symmetric chain carbonate of Formula (5) relative to the composition is preferably 0.01-10 mass%, more preferably 0.05-5 mass-%, still more preferably 0.1-2 mass-%.

The amounts of the symmetric or asymmetric chain carbonates of Formulae (3)-(5) can be measured by gas chromatography.

A further composition that can be produced by the present method includes an asymmetric chain carbonate of the Formula R¹OCOOR² (3), wherein R¹ is an organic group; and R² is an organic group different from R¹, and water, wherein the content of water in the composition is ≤ 100 ppm.

The amount of water can be measured using a Karl Fischer moisture meter.

### EXAMPLES

The present invention will be specifically described below with reference to, but not limited to, examples.

### Example 1

Stirring of 2,2,2-trifluoroethanol (1 g, 10 mmol), calcium oxide (560 mg, 10 mmol), and dichloromethane (2.84 g, 30 mmol) was performed at 0°C to 5°C. To the resulting mixture was carefully dropwise added methyl chloroformate (0.992 g, 10.5 mmol) while preventing heat generation. After the completion of the dropping, the mixture was stirred for about 16 hours while gradually raising the temperature to room temperature, and then filtrated and distillated, thereby producing a composition containing CF₃CH₂OCOOCH₃ and CH₃OCOOCH₃ with a conversion rate of 99.0%. The conversion rate means the conversion rate of the alcohol (2,2,2-trifluoroethanol in Example 1) determined by gas chromatography (GC). Table 1 shows the result. Regarding the water content of the system, the composition produced by the reaction was pulled out with a dry syringe and subjected to measurement using a Karl Fischer moisture meter to confirm that the composition contained water in an amount of 22 ppm (average of three measures).

### Example 2

A composition containing CF₃CH₂OCOOCH₃ and CH₃OCOOCH₃ was produced as in Example 1, except that calcium hydroxide was used instead of calcium oxide. The conversion rate was 99.9%. The water content was measured using a Karl Fischer moisture meter and confirmed to be 85 ppm (average of three measures).

### Example 3

A composition containing CF₃CH₂OCOOCH₃ and CH₃OCOOCH₃ was produced as in Example 1, except for using no dichloromethane. The conversion rate was 95.4%.

### Comparative Example 1

The same operation as in Example 1 was performed, except that pyridine was used instead of calcium oxide. The conversion rate was only 24.0%. The methyl chloroformate was decomposed. A lot of symmetric carbonate was observed.

### Example 4

A composition containing CF₃CH₂OCOOCH₃ and CH₃OCOOCH₃ was produced as in Example 1, except that methanol, trifluoroethyl chloroformate, and calcium hydroxide were used instead of 2,2,2-trifluoroethanol, methyl chloroformate, and calcium oxide, respectively. The conversion rate was 99.6%.

### Example 5

A composition containing CH₃CH₂OCOOCH₃ and CH₃OCOOCH₃ was produced as in Example 1, except that ethanol and calcium hydroxide were used instead of 2,2,2-trifluoroethanol and calcium oxide, respectively. The conversion rate was 99.3%.

### Example 6

A composition containing CH₃OCH₂CH₂OCOOCH₃ and CH₃OCOOCH₃ was produced as in Example 1, except that 2-methoxy ethanol and calcium hydroxide were used instead of 2,2,2-trifluoroethanol and calcium oxide, respectively. The conversion rate was 99.4%.

### Example 7

A composition containing C₆H₅OCOOCH₃ and CH₃OCOOCH₃ was produced as in Example 1, except that phenol and calcium hydroxide were used instead of 2,2,2-trifluoroethanol and calcium oxide, respectively. The conversion rate was 99.0%.

### Example 8

Stirring of 2,2,2-trifluoroethanol (1 g, 10 mmol), potassium carbonate (1.38 g), calcium chloride (1.11 mg, 10 mmol), and dichloromethane (2.84 g, 30 mmol) was performed at 0°C to 5°C. To the resulting mixture was carefully dropwise added methyl chloroformate (0.992 g, 10.5 mmol) while preventing heat generation. After the completion of the dropping, the mixture was stirred for about 16 hours while gradually raising the temperature to room temperature, and then filtrated and distillated, thereby producing a composition containing CF₃CH₂OCOOCH₃ and CH₃OCOOCH₃ with a conversion rate of 94.8%.

### Comparative Example 2

The same operation as in Example 4 was performed, except that pyridine was used instead of calcium hydroxide. The conversion rate was only 34.3%. The trifluoroethyl chloroformate was decomposed. A lot of symmetric carbonate was observed.

### Comparative Example 3

The same operation as in Example 5 was performed, except that pyridine was used instead of calcium hydroxide. The conversion rate was only 36.6%. The methyl chloroformate was decomposed. A lot of symmetric carbonate was observed.

### Comparative Example 4

The same operation as in Example 8 was performed, except for adding no calcium chloride. The conversion rate was only 31.4%. The methyl chloroformate was decomposed. A lot of symmetric carbonate was observed.

The selectivity in the following table was determined based on the area ratios measured by gas chromatography. In the table "Ex." = Example and "CE" = Comparative Example.

**[Table 1]**

| | GC area % | | | | Conversion rate % | Selectivity % |
|---|---|---|---|---|---|---|
| | R¹OH | CH₃OCOCl | CH₃OCOOCH₃ | R¹OCOOCH₃ | | |
| Example 1 | 0.98 | 0.91 | 1.42 | 96.69 | 99.0 | 98.6 |
| Example 2 | 0.12 | 1.11 | 1.47 | 97.30 | 99.9 | 98.5 |
| Example 3 | 4.55 | 0.74 | 0.85 | 93.86 | 95.4 | 99.1 |
| Example 4 | 0.35 | 0.88 | 1.39 | 96.88 | 99.6 | 98.5 |
| Example 5 | 0.69 | 2.08 | 1.51 | 95.72 | 99.3 | 98.4 |
| Example 6 | 0.55 | 1.82 | 1.43 | 96.20 | 99.4 | 98.5 |
| Example 7 | 0.99 | 1.67 | 1.88 | 95.72 | 99.0 | 98.1 |
| Example 8 | 4.20 | 17.17 | 1.38 | 77.25 | 94.8 | 98.2 |
| Comparative Example 1 | 57.33 | 0.00 | 24.55 | 18.12 | 24.0 | 42.4 |
| Comparative Example 2 | 48.78 | 0.00 | 25.80 | 25.42 | 34.3 | 49.6 |
| Comparative Example 3 | 50.18 | 0.00 | 20.89 | 28.99 | 36.6 | 58.1 |
| Comparative Example 4 | 49.24 | 0.00 | 28.17 | 22.59 | 31.4 | 44.5 |

## Claims

1. A method for producing an asymmetric chain carbonate, comprising reacting an alcohol of formula (1):
R¹OH (1)
wherein R¹ is an organic group, with a halocarbonate ester compound of formula (2):
XCOOR² (2)
wherein X is a halogen atom; and R² is an organic group different from R¹, in the presence of a basic magnesium salt, or a basic alkaline earth metal salt, or an inorganic base and a desiccant, to produce an asymmetric chain carbonate of formula (3):
R¹OCOOR² (3)
wherein R¹ and R² are as defined above.

2. The method of claim 1, wherein R¹ and R² each independently are alkyl optionally having a fluorine atom.

3. The method of claim 1 or 2, wherein the reaction is carried out in the presence of an inorganic base and a desiccant, and the inorganic base is at least one of carbonates of alkali metals, hydroxides of alkali metals, oxides of magnesium, oxides of alkaline earth metals, hydroxides of magnesium, hydroxides of alkaline earth metals, carbonates of magnesium, carbonates of alkaline earth metals, acetates of magnesium, and acetates of alkaline earth metals.

4. The method of any of claims 1-3, wherein the reaction is carried out in the presence of an inorganic base and a desiccant, and the desiccant is at least one of neutral calcium salts, zeolite, silica gel, alumina, and activated carbon.

## Patentansprüche

1. Verfahren zur Herstellung eines asymmetrischen Kettencarbonats, umfassend das Umsetzen eines Alkohols der Formel (1):
R¹OH (1)
worin R¹ eine organische Gruppe ist, mit einer Halocarbonatesterverbindung der Formel (2):
XCOOR² (2)
worin X ein Halogenatom ist; und R² eine organische Gruppe ist, die sich von R¹ unterscheidet, in der Gegenwart eines basischen Magnesiumsalzes, oder eines basischen Erdalkalimetallsalzes, oder einer anorganischen Base und eines Trockenmittels, um ein asymmetrisches Kettencarbonat der Formel (3) herzustellen:
R¹OCOOR² (3)
worin R¹ und R² wie vorstehend definiert sind.

2. Verfahren gemäß Anspruch 1, worin R¹ und R² jeweils unabhängig Alkyl sind, optional aufweisend ein Fluoratom.

3. Verfahren gemäß Anspruch 1 oder 2, worin die Umsetzung in der Gegenwart einer anorganischen Base und eines Trockenmittels durchgeführt wird und die anorganische Base zumindest eine ist von Carbonaten der Alkalimetalle, Hydroxiden der Alkalimetalle, Oxiden von Magnesium, Oxiden von Erdalkalimetallen, Hydroxiden von Magnesium, Hydroxiden von Erdalkalimetallen, Carbonaten von Magnesium, Carbonaten von Erdalkalimetallen, Acetaten von Magnesium und Acetaten von Erdalkalimetallen ist.

4. Verfahren gemäß irgendeinem der Ansprüche 1-3, worin die Umsetzung in der Gegenwart einer anorganischen Base und eines Trockenmittels durchgeführt wird und worin das Trockenmittel zumindest eines ist von neutralen Calciumsalzen, Zeolithe, Silikagel, Aluminiumoxid und Aktivkohle.

## Revendications

1. Procédé de production d'un carbonate à chaîne asymétrique, comprenant une réaction d'un alcool de formule (1) :
R¹OH (1)
dans lequel R¹ est un groupe organique, avec un composé d'ester d'halogénocarbonate de formule (2) :
XCOOR² (2)
dans lequel X est un atome d'halogène ; et R² est un groupe organique différent de R¹, en présence d'un sel de magnésium basique, ou d'un sel de métal alcalino-terreux basique, ou d'une base inorganique et d'un agent dessiccatif, pour produire un carbonate à chaîne asymétrique de formule (3) :
R¹OCOOR² (3)
dans lequel R¹ et R² sont tels que définis précédemment.

2. Procédé selon la revendication 1, dans lequel R¹ et R² sont chacun indépendamment un alkyle présentant facultativement un atome de fluor.

3. Procédé selon la revendication 1 ou 2, dans lequel la réaction est réalisée en présence d'une base inorganique et d'un agent dessiccatif, et la base inorganique est au moins certains parmi des carbonates de métaux alcalins, des hydroxydes de métaux alcalins, des oxydes de magnésium, des oxydes de métaux alcalino-terreux, des hydroxydes de magnésium, des hydroxydes de métaux alcalino-terreux, des carbonates de magnésium, des carbonates de métaux alcalino-terreux, des acétates de magnésium, et des acétates de de métaux alcalino-terreux.

4. Procédé selon l'une quelconque des revendications 1-3, dans lequel la réaction est réalisée en présence d'une base inorganique et d'un agent dessiccatif, et l'agent dessiccatif est au moins un parmi des sels de calcium neutre, une zéolite, un gel de silice, une alumine, et un charbon actif.
